# EUROPÄISCHE PATENTSCHRIFT

(11) **EP 0 514 435 B1**
(45) Veröffentlichungstag und Bekanntmachung des Hinweises auf die Patenterteilung: **06.10.1993**
(21) Anmeldenummer: 91903667.3
(22) Anmeldetag: 06.02.1991
(51) Int. Cl.: A61K 9/127, A61K 9/06, A61K 7/48

(54) **ALKOHOL ENTHALTENDE WÄSSRIGE GELARTIGE PHOSPHOLIPIDZUSAMMENSETZUNG, IHRE VERWENDUNG UND TOPISCHE ZUBEREITUNGEN, DIE DIESE ENTHALTEN**
ALCOHOLIC AQUEOUS GEL-TYPE PHOSPHOLIPID COMPOSITION, ITS USE AND TOPICAL PREPARATIONS CONTAINING IT
COMPOSITION PHOSPHOLIPIDIQUE AQUEUSE SOUS FORME DE GEL A TENEUR EN ALCOOL, SON UTILISATION ET PREPARATIONS TOPIQUES LA RENFERMANT

(30) Priorität: 08.02.1990 DE 4003782; 08.02.1990 DE 4003783
(43) Veröffentlichungstag der Anmeldung: 25.11.1992
(73) Patentinhaber: A. Nattermann & Cie. GmbH, D-50829 Köln (DE)
(72) Erfinder: GHYCZY, Miklos, D-5000 Köln 41 (DE); RÖDING, Joachim, D-5000 Köln 30 (DE); LAUTENSCHLÄGER, Hans, D-5024 Pulheim (DE); HAMEISTER, Walter, D-5024 Pulheim (DE); HAGER, Jörg, D-5000 Köln 30 (DE)
(74) Vertreter: Döring, Wolfgang, Dr.-Ing. Patentanwälte Hauck, Graalfs, Wehnert, Döring, Siemons
(86) Internationale Anmeldenummer: EP9100229
(87) Internationale Veröffentlichungsnummer: WO9111993

(56) Entgegenhaltungen:
- EP-A- 0 051 833
- EP-A- 0 069 307
- EP-A- 0 100 459
- WO-A-90/09782

## Beschreibung

Die vorliegende Erfindung betrifft eine Alkohol enthaltende wäßrige gelartige Phospholipidzusammensetzung sowie ihre Verwendung. Die vorliegende Erfindung betrifft weiterhin topisch applizierbare Zubereitungen, die diese enthalten.

Gele sind formbeständige, leicht deformierbare, an Flüssigkeiten reiche disperse Systeme aus mindestens zwei Komponenten. Die am meisten bekannten und weit verbreitetsten Gele sind die wäßrigen Gele, in denen Wasser als stets vorhandene Hauptkomponente verwendet wird. Bekannt sind ferner die sogenannten Organogele, in denen die flüssige Hauptkomponente ein organisches Lösemittel ist und ein zugesetztes apolares Polymer, die Gelierung verursacht und ihre Stärke beeinflußt.

So beschreibt die WO 86/02264 ein System aus umgekehrten Micellen, das durch Zugabe von geeigneten Lösemitteln, wie beispielsweise Squalen, Miglyol® oder Pflanzenölen in entsprechende Gele überführt werden kann.

Es wurden auch Systeme untersucht, in denen Gele aus Lecithin oder allgemeinen Phospholipiden, einem Lösemittel, Wasser oder weiteren benötigten Hilfsstoffen gebildet werden. Solche Gele dienen oft jedoch nur als Zwischenstufe, um daraus als gewünschtes Endprodukt Liposomendispersionen zu bilden.

Liposome sind kugelförmige Vesikel mit einer Hülle aus einer oder mehreren Doppelschichten (Bilayer). Sie werden bevorzugt aus Lipiden natürlicher Herkunft erzeugt. In der pharmazeutischen Industrie und auf dem Gebiet der Kosmetik spielen besonders die Liposome aus Phospholipiden eine entscheidende Rolle. Die wichtigsten Phospholipidquellen sind die Sojabohne und andere phospholipidreiche Pflanzen, beispielsweise Raps oder Erdnuß, in geringerem Maße werden die Phospholipide auch aus dem Ei oder von Tieren gewonnen.

Unter bestimmten Voraussetzungen sind Phospholipide in der Lage, in wäßriger Lösung Liposome zu bilden (Bangham, A.D., Horne, R.W., J. Mol. Biol. 8 (1964), S. 660 ff.). Seitdem ist auf zahlreiche Art und Weise versucht worden, stabile Liposome, die für eine breite Anwendung Möglichkeiten bieten, herzustellen. Die von Bangham (Bangham, A.D., et al., Meth. in Membrane Biol. 1 (1976), S. 1-68) vorgeschlagene Methode, Phospholipide in einem organischen Lösemittel aufzulösen, letzteres im Rotationsverdampfer zu entfernen, um auf der Kolbenwand einen Lipidfilm zu erhalten, der dann beim Dispergieren in Wasser oder wäßriger Lösung Liposome ausbildet, ist im technischen Maßstab nicht durchführbar. Die so hergestellten Liposome sind nur für einen kleinen Anwendungsbereich zu verwenden.

Liposome können durch die Beschallung von multilamellaren Liposomen, wie sie etwa nach der obigen Methode erhalten werden können, mittels Ultraschall in kleinere, unilamellare Vesikel umgesetzt werden (C. Huang, Biochemistry 8 (1969), S. 346-352).

Unilamellare Liposomen können ebenfalls mittels der "French Press" bei niederen Drucken hergestellt werden, indem auf übliche Weise hergestellte multilamellare Liposomenpräparate durch eine enge Öffnung gepreßt werden (Hamilton, R.L., et al., J. Lipid Res. 21 (1980), S. 981-992).

Eine andere Möglichkeit zur Erzeugung liposomaler Lösungen stellt die Ethanol-Injektionsmethode von Batzri und Korn dar (Batzri, S., Korn, E.D., Biochim. Biophys. Acta 298 (1976), S. 1015-1019). Hierbei wird das in Ethanol gelöste Lipid in eine wäßrige Pufferlösung injiziert, so daß sich Liposome bilden. Dieses Verfahren ist, genau wie die Filmmethode von Bangham, nicht in technischem Maßstab durchführbar. Bei beiden Methoden muß außerdem das organische - möglicherweise sogar toxische - Lösemittel aufwendig entfernt werden, um pharmazeutisch oder kosmetisch anwendbare Präparate zu erhalten.

Große unilamellare Liposome werden auch gebildet, wenn geladene Lipide in einem Puffer in Gegenwart von Calcium-Kationen suspendiert werden (Papahadjopoulos, D., Ann. N.Y. Acad. Sci. 308 (1978) S. 751). Nach Entfernung der Calcium-Kationen bilden sich dann große unilamellare Liposomen.

Eine neuere Methode zur Bildung von Liposomen besteht darin, Phospholipide in einem kationischen Detergens in ein organisches Lösemittel zu geben und das Lipidgemisch auf eine fein verteilte bzw. fein strukturierte Oberfläche wie Molekularsiebe, Quarz oder Zeolithe zu bringen (D.D. Lasic, J. of Coll. and Interface Sci. 124 82), (1988), S. 428-435) und anschließend das Lösungsmittel im Vakuum zu entfernen. Dabei entstehen direkt Phospholipid-Vesikel.

Eine allgemeine Übersicht über die gebräuchlichsten Methoden zur Liposomen-Herstellung geben z.B. die Artikel von Szoka, F. et al. in Ann. Rev. Biophys. Bioeng. 9 (1980), S. 467-508 und Lasic, D.D. in Biochem. J. 256 (1988), S. 1-11.

In der EP-A-0 160 266 wird eine Liposomenzusammensetzung beansprucht, die aus einem dreidimensionalen Netzwerk von Liposomen und einem Netzwerkmaterial besteht. Für das Netzwerkmaterial werden bevorzugt Polysaccharide verwendet, in dem die Liposome eingebettet sind.

Nach der WO 85/03640 werden beladene Liposome in einer Gelmatrix aus Stärke oder modifizierter Stärke beansprucht.

In der EP-A-0 069 307 wird eine Methode zur Darstellung eines Liposomengels beschrieben, wonach eine wäßrige oder lösemittelhaltige Lecithinlösung mit Ultraschall behandelt wird. Abhängig von der Beschallungsdauer und Beschallungsintensität bildet sich ein mehr oder weniger viskoses Gel aus. Durch Verlängerung der Beschallungszeit oder mittels mechanischer Rührwirkung erhält man als Endprodukt eine Liposomen enthaltende, wäßrige Lösung.

Ein sogenanntes Preliposomengel erhält man aus einem Gemisch von Phospholipiden, Fettsäuren und einem hydratisierenden Agens gemäß EP-A-0 211 647. Nach Zugabe von Wasser oder Pufferlösung bilden sich Liposome aus.

Ein phospholipidhaltiges, dünnflüssiges Gel wird in der WO 89/00077 für die Anwendung als Aerosol-Liposome beansprucht. Das System besteht aus Lecithin, einem organischen Lösemittel und wenig Wasser. Hier tritt eine weite Spanne von Liposomendurchmessern im Bereich von 100-2500 nm auf; für einen sinnvollen Anwendungsbereich muß ein hoher Lösungsmittelanteil gewählt werden.

Die US-A-2,090,537 betrifft ein Verfahren zur Herstellung von "wasserhaltigem" Lecithin (Lecithinhydrat), bestehend aus einer homogenen Mischung von vorzugsweise 15-25% pflanzlichem Lecithin, vorzugsweise 8-25% Alkohol, insbesondere Ethanol oder Isopropanol, sowie 58-78% Wasser als Rest. Das wasserhaltige Lecithin wird erhalten, indem man Wasser und Alkohol vorzugsweise auf etwa 71°C (160°F) erhitzt, das Lecithin hinzufügt und umrührt. Nach dem Abkühlen auf Raumtemperatur kommt es zu einer Phasentrennung, bei der die unterste Phase der drei Phasen das Lecithinhydrat enthält. Diese Phase aus Lecithin, gesättigt mit Alkohol, Wasser und Öl, ist bereits als solche hinreichend stabil und kann weiter gereinigt werden durch Entfernung des Alkohols oder eines Teiles des Wassers im Vakuum, wobei man das wasserhaltige Lecithin erhält. Alternativ kann man diese Phase durch Ansäuern auf PH4 bis PH6 als Gel erhalten.

Die EP-A-0 158 441 betrifft eine flüssige Zusammensetzung, enthaltend eine homogene Mischung von wenigstens einem Membranlipid, wenigstens einem wassermischbaren organischen Lösemittel, z.B. Ethanol oder Propylenglycol, das als Lösemittel für das Lipid dient und gegebenenfalls eine Menge Wasser, die dadurch gekennzeichnet ist, daß diese Zusammensetzung bei Zugabe von zusätzlichem Wasser spontan Vesikel oder Liposome bildet, wobei das Gewichtsverhältnis Lipid:Lösemittel 40:1 bis 1:20 beträgt.

Die EP-A-0 240 346 beschreibt ein Herstellverfahren für Liposome mit vergrößertem Reservoir für Wirkstoffe mit folgenden Verfahrensschritten:
1. Herstellung eines Liposoms mit und ohne ein Wirkstoffreservoir aus einem Phospholipid;
2. Dispergieren des Liposoms in einer wirkstoffhaltigen Flüssigkeit;
3. Hinzufügen eines leicht flüchtigen organischen Lösemittels zur Dispersion unter Gelbildung; und dann
4. Entfernen dieses organischen Lösemittels durch Abdampfen und Wiederherstellen der Liposome.

Die Liposome nach Verfahrensschritt 1 werden entweder als multilamellare Liposomen in an sich bekannter Weise oder als unilamellare Liposomen durch Ultraschallbehandlung erhalten. Die Liposomen nach Verfahrensschritt 4 erhalten nach ihrer Aufarbeitung keine wesentlichen Anteile an organischen Lösemitteln mehr.

Der vorliegenden Anmeldung liegt die Aufgabe zugrunde, eine Alkohol enthaltende, wäßrige gelartige Phospholipidzusammensetzung zu schaffen, die selbstkonservierend, lagerfähig und transparent ist.

Diese Aufgabe wird dadurch gelöst, daß die gelartige Phospholipidzusammensetzung ein liposomales Gel ist, d.h. ein ausschließlich aus Liposomen aufgebautes System, das aus einem speziell zusammengesetzten Phospholipidkonzentrat, Alkohol und Wasser in speziellen Konzentrationen besteht und dessen wäßrige Phase praktisch ausschließlich innere Phase ist.

Die Erfindung betrifft somit eine Alkohol enthaltende wäßrige gelartige Phospholipidzusammensetzung, die als Alkohol Ethanol, 1-Propanol, 2-Propanol oder deren Mischungen enthält, die dadurch gekennzeichnet ist, daß die Phospholipidzusammensetzung ein liposomales Gel mit folgender Zusammensetzung ist:
- 15,00 - 30,00 Gew.Tl. eines Phospholipidkonzentrats, bestehend aus
   70,00 - 80,00 Gew.Tl. Phosphatidylcholin,
   15,00 - 5,00 Gew.Tl. sauren Phospholipiden,
   5,00 - 25,00 Gew.Tl. sonstigen Phospholipiden,
   wobei dieses Konzentrat pro 100 Gew.Tl. vorstehender Phospholipide weiterhin 1 - 15 Gew.Tl. phosphorfreie Begleitlipide enthält,
- 20,00 - 14,00 Gew.Tl. Alkohol und
- 50,00 - 71,00 Gew.Tl. einer wäßrigen Lösung.

Die erfindungsgemäße gelartige Phospholipidzusammensetzung weist eine transparente Struktur auf, die homogen ist und weitgehend frei von Agglomerationen und sonstigen Trübungen ist und eine mittlere Teilchengröße von 200 nm ± 20% aufweist. (Elektronenmikroskopie, Gefrierbruch). Die aus der gelartigen Phospholipidzusammensetzung durch Verdünnen mit wäßriger Lösung erhaltene liposomale Lösung weist vorzugsweise eine durchschnittliche Größe der Liposomen von 200 nm ± 20% (bestimmt nach der Laserlichtstreuungsmethode) auf und ist somit bevorzugt in topischen Zubereitungen, wie kosmetischen oder pharmazeutischen Zubereitungen, einzusetzen, die für Liposome einen Teilchendurchmesser von 100 - 400 nm, vorzugsweise 100 - 200 nm, voraussetzen. Ein besonderer Vorteil ist, daß diese Liposome nicht nur im unbeladenen, sondern auch im beladenen Zustand wirkstoffabhängig transparent bleiben. Weiter sind sowohl das Gel wie auch die liposomale Lösung steril und pyrogenfrei, nach DAB 9, herstellbar, so daß sie ohne zusätzliche, gegebenenfalls allergene Konservierungsstoffe zu kosmetischen und pharmazeutischen Zubereitungen formuliert werden können. Weiterhin ist es für den Fachmann überraschend gewesen, daß Alkohol in Konzentrationen von 14 bis 20 Gew.% nicht zu einer Zerstörung der Liposomenlösung führt.

Schließlich kann aus der erfindungsgemäßen Phospholipidzusammensetzung (Liposomengel) eine liposomale Lösung in technisch einfacher Weise erhalten werden, ohne technisch und energetisch aufwendige Verfahrensschritte durchführen zu müssen, also insbesondere ohne die Temperatur zu erhöhen oder Ultraschall einzusetzen.

Die Phospholipidkonzentrate als einer der Bestandteile der erfindungsgemäßen Phospholipidzusammensetzung werden aus natürlichen Phospholipidgemischen erhalten, beispielsweise aus Ölsaaten, wie Sojabohne, Raps, Sonnenblume usw.

Ein Anreicherungsverfahren zur Herstellung derartiger Phospholipidkonzentrate ist in der EP-A-0 069 770 beschrieben. Derartige Phospholipidkonzentrate bestehen aus Phospholipiden (Phosphatidylcholin, sauren Phospholipiden und sonstigen Phospholipiden) sowie phosphorfreien Begleitlipiden. Zu den sauren Phospholipiden zählt das Phosphatidylethanolamin, die Phosphatidsäure sowie das N-Acyl-Phosphatidylethanolamin. Zu den sonstigen Phospholipiden zählt das Lyso-Phosphatidylcholin und das Phosphatidylinosit. Zu den phosphorfreien Begleitlipiden zählen u. a. die Glycolipide und die Phytolipide. Die phosphorfreien Begleitlipide liegen in den Phospholipidkonzentraten, bezogen auf 100 Gew.Tl. Phospholipide, in 1-15 Gew.Tl., vorzugsweise 1-9 Gew.Tl., besonders bevorzugt 1-5 Gew.Tl., vor.

Das vorstehend beschriebene Phospholipidkonzentrat weist somit folgende Zusammensetzung auf:
60,87 - 79,21 Gew.-% Phosphatidylcholin
14,85 - 4,35 Gew.-% saure Phospholipide
4,95 - 21,74 Gew.-% sonstige Phospholipide und
0,99 - 13,04 Gew.-% phosphorfreie Begleitlipide.

Eine weitere bevorzugte Ausführungsform des Phospholipidkonzentrats als Bestandteil der erfindungsgemäßen Phospholipidzusammensetzung ist eine Mischung aus 80 Gew.Tl. Phosphatidylcholin, 5-15 Gew.Tl. sauren Phospholipiden, und 15-5 Gew.Tl. sonstigen Phospholipiden, wobei dieses Konzentrat pro 100 Gew.Tl. vorstehender Phospholipide weiterhin 1-9 Gew.Tl. phosphorfreie Begleitlipide enthält. Ein derartiges bevorzugtes Phospholipidkonzentrat weist somit folgende Zusammensetzung auf:
73,39 - 79,21 Gew.% Phosphatidylcholin,
4,95 - 13,76 Gew.% saure Phospholipide,
14,85 - 4,59 Gew.% sonstige Phospholipide und
0,99 - 8,26 Gew.% phosphorfreie Begleitlipide.

Das Phospholipidkonzentrat wird in Mengen von 15,00 - 30,00 Gew.Tl., vorzugsweise 20,20 - 30,00 Gew.Tl./100 Gew.Tl. der erfindungsgemäßen Phospholipidzusammensetzung eingesetzt.

Der Alkohol wird in Mengen von 14 - 20 Gew.Tl., vorzugsweise etwa 16 Gew.Tl. pro 100 Gew.Tl. der erfindungsgemäßen Phospholipidzusammensetzung eingesetzt.

Die wäßrige Lösung wird in Mengen von 50 - 71,00 Gew.Tl., vorzugsweise 54,00 - 63,80 Gew.Tl., pro 100 Gew.Tl. der Phospholipidzusammensetzung eingesetzt. Unter wäßriger Lösung im Sinne der vorliegenden Erfindung wird einmal destilliertes Wasser, Leitungswasser, gereinigtes Wasser DAB 9, entmineralisiertes Wasser, wie auch Pufferlösungen, wie etwa Phosphatpuffer oder eine physiologische Kochsalzlösung verstanden.

Bei der durch Verdünnen der Phospholipidzusammensetzung unter Rühren erhaltene liposomale Lösung liegen die Bestandteile in folgenden Konzentrationen vor:

Das Phospholipidkonzentrat liegt in der liposomalen Lösung in Mengen von 10,10 - 20,20 Gew.Tl., bevorzugt 10,10 Gew.Tl., bezogen auf 100 Gew.Tl. der gesamtlimposomalen Lösung vor. Der Alkohol liegt in der liposomalen Lösung in Mengen von etwa 16 6ew.Tl., bezogen auf 100 Gew.Tl. der gesamtliposomalen Lösung vor. Die wäßrige Lösung liegt in der liposomalen Lösung in Mengen von 63,80 - 73,90 Gew.Tl., bezogen auf 100 Gew.Tl. der gesamtliposomalen Lösung vor. Nach einer bevorzugten Ausführungsform der vorliegenden Erfindung kann dem liposomalen Gel wenigstens ein biologisch aktiver Wirkstoff zugemischt werden. Beispiele für derartige Wirkstoffe sind Antiphlogistika wie Ketoprofen, Bisabolol usw., Anticoagulantika wie Heparin, Hirudin usw., Antimykotika, sowie Spasmolytika oder durchblutungsfördernde Mittel wie Papaverin.

Die vorliegende Erfindung betrifft weiterhin topisch applizierbare Zubereitungen, die wenigstens eine der vorstehend beschriebenen Phospholipidzusammensetzungen in Kombination mit wengistens einem biologisch aktiven Wirkstoff und üblichen Hilfs- und Zusatzstoffen enthält. Biologisch aktive Wirkstoffe, die im Zusammenhang mit Gelen verabreicht werden sollen, sind beispielsweise vorstehend beschriebene Wirkstoffe.

Die vorliegende Erfindung betrifft weiterhin pharmazeutische Zubereitungen, die wenigstens eine Phospholipidzusammensetzung der vorstehend beschriebenen Art in Kombination mit wenigstens einem biologisch aktiven Wirkstoff enthalten, vorzugsweise zur Behandlung der vorstehend beschriebenen Indikationen.

Die vorliegende Erfindung betrifft schließlich kosmetische Zubereitungen, die wenigstens eine vorstehend beschriebene Phospholipidzusammensetzung in Kombination mit wenigstens einem kosmetischen Wirkstoff zur Haut- und Haarpflege enthält, wobei es sich vorzugsweise um ein in die Hornhaut penetrierendes pflegendes Mittel, wie beispielsweise Harnstoff, Elastin usw., handelt.

Eine besonders bevorzugte Ausführungsform der vorliegenden Erfindung ist ein Gel, das aus 20 % Phospholipid (mit einem Gehalt von 80 % Phosphatidylcholin) und 16 % Ethanol besteht und welches durch folgende Parameter bestimmt wird:
- Aussehen gold-braunes, leicht trübes Gel
- Transmission (c= 0,5 % in Wasser, 660 nm) mind. 50% (DAB 9, V.6.19)
- Viskosität (gem. bei 20°C) 5000 ± 2000 mPa.s

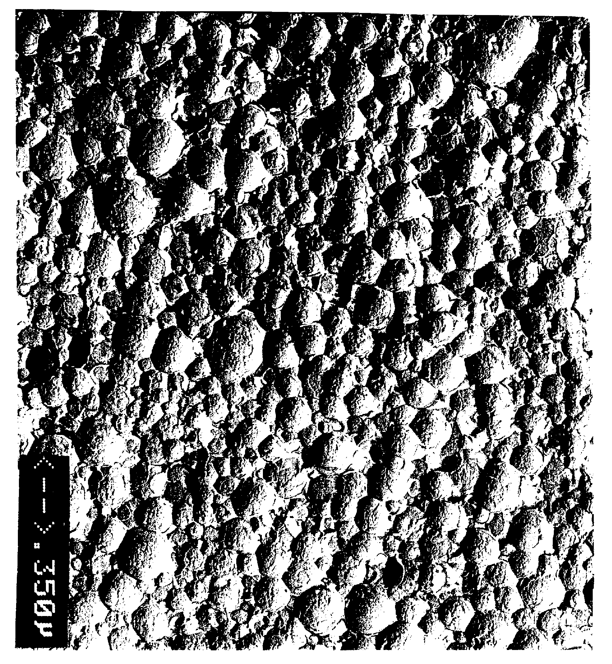

Die elektronenmikroskopische Untersuchung nach der Methode von Müller, T. et al., Seifen Öle Fette Wachse 3, 88-89 (1989), zeigt nach Anwendung der Gefrierbruchtechnik die liposomale Struktur des Gels (Fig. 1).

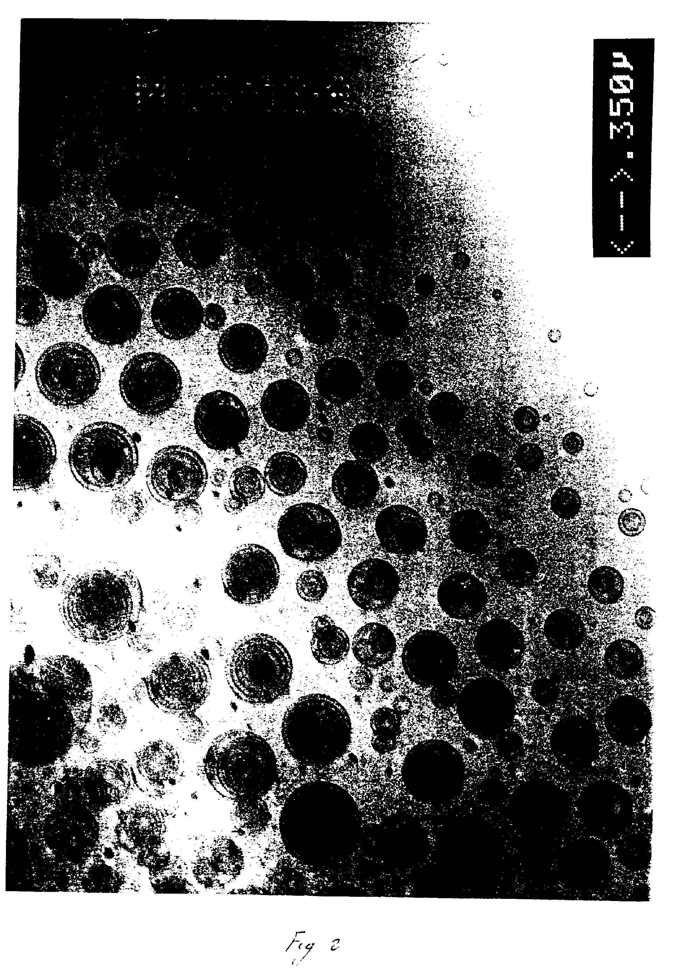

Der Nachweis der Liposome erfolgte nach der von Zellmann et al., ZEISS Application EM 902 Cryo, 1989, beschriebenen Methode (Fig. 2).

Die vorliegende Erfindung wird nachfolgend anhand von 2 Figuren, die bevorzugte Ausführungsformen der Erfindung zeigen, näher erläutert.

Es zeigen:
- Fig. 1:: Gezeigt werden die Liposomen der nach der Gefrierbruchtechnik präparierten Probe des erfindungsgemäß hergestellten Gels in Form einer elektronenmikroskopischen Aufnahme. Die Liposomen bilden ein vesikuläres Gel. Sie haben engen Kontakt untereinander und es kann kein zusätzliches Wasser gesehen werden. (1 Maßeinheit entspricht 350 nm).
- Fig. 2:: Die kryoelektronenmikroskopische Aufnahme einer 3%-igen Dispersion des erfindungsgemäß hergestellten Gels in Wasser zeigt, daß eine solche Präparation ausschließlich multilamellare Liposomen enthält. (1 Maßeinheit entspricht 350 nm).

Die Herstellung der gelartigen Phospholipidzusammensetzung erfolgt in technisch besonders einfacher Art und Weise, indem man das Phospholipidkonzentrat bestimmter Zusammensetzung mit einer bestimmten Menge an Alkohol kurze Zeit rührt und durch Wasserzugabe und weiteres Rühren Gelbildung veranlaßt. Das Durchrühren kann mit jedem handelsüblichen Rührer durchgeführt werden.

Allerdings muß dieser eine genügend große Drehzahl besitzen, so daß in kurzer Zeit eine starke Durchmischung erreicht wird. Hierbei ist von einer Phospholipidzusammensetzung auszugehen, die im allgemeinen einen pH-Wert im Bereich von 5 bis 8, vorzugsweise 6,5 bis 7,5 aufweist.

Die Erfindung wird nun anhand von Ausführungsbeispielen näher erläutert, wobei folgende Phospholipidkonzentratzusammensetzung verwendet wird:

Die Phospholipidanteile dieser Zusammensetzung bestehen aus:
Phosphatidylcholin 80 %;
saure Phospholipide 15 %;
sonstige Phospholipide 5 %.

Die phosphorfreien Begleitlipide liegen bei 5 Gew.Tl. bezogen auf 100 Gew.Tl. Phospholipide, d.h. es sind 4,76 Gew.% phosphorfreie Begleitlipide enthalten.

### Beispiel 1

10,48 g des Phospholipidkonzentrates (mit 10 g Phospholipiden) werden in 8 g Ethanol unter Rühren aufgelöst. Die Lösung besitzt eine Viskosität von 806 mPa.s (bei 25°C) und ist homogen. Die Lösung wird mit 31,52 g entmineralisiertem Wasser während 3 Min. homogenisiert, wobei ein handelsüblicher schnellaufender Laborrührer verwendet wird. Man erhält ein transparentes Gel mit 20,96 Gew.% Phospholipidkonzentrat (20 Gew.-% Phospholipiden).

### Beispiel 2

Analog Beispiel 1 werden 15,72 g des Phospholipidkonzentrates (mit 15 g Phospholipid) in 8 g Ethanol gelöst. Die Lösung wird mit 26,28 g entmineralisiertem Wasser 3 Min. gerührt, bis ein homogenes, transparentes Gel entsteht. Das Gel enthält 31,4 Gew.% Phospholipidkonzentrat (30 Gew.% Phospholipide).

### Beispiel 3

Analog Beispiel 1 werden 15,72 g des Phospholipidkonzentrates (mit 15 g Phospholipid) in 8 g 2-Propanol gelöst. Es werden 26,28 g entmineralisiertes Wasser zugegeben und noch 3 Min. gerührt. Es entsteht ein transparentes Gel, das 31,40 Gew.% Phospholipidkonzentrat (30,00 Gew.Tl. Phospholipide) enthält.

### Beispiel 4

Wie in Beispiel 1 werden 10,48 g des Phospholipidkonzentrates (mit 10 g Phospholipiden) in 8 g 2-Propanol gelöst. Nach Zugabe von 36,52 g entmineralisiertem Wasser wird 3 Min. gerührt und man erhält ein transparentes Gel mit 19,05 Gew.% Phospholipidkonzentrat (18,18 Gew.% Phospholipiden).

Die nachfolgenden Beispiele zeigen, wie durch einfache Verfahrensschritte aus dem phospholipidhaltigen liposomalen Gel liposomale Lösungen erhalten werden.

### Beispiel 5

Die gesamte Menge des in Beispiel 1 erhaltenen Phospholipidgels (50 g) wird mit 42 g 0,2 molarer Phosphat-Pufferlösung pH 7,4 versetzt und 4 Min. gerührt. Die entstehende dünnflüssige Dispersion wird mit 8 g Ethanol versetzt und noch eine Minute bis zum fertigen Endprodukt weitergerührt. Die Verhältnisse Phospholipidkonzentrat: Ethanol:wäßriger Lösung betragen 20,96:16:73,04 (Phospholipid:Ethanol:Wasser betragen 10:16:74). Die mittlere Teilchengröße, gemessen nach der Laserstreulichtmethode, beträgt 204 nm (± 20%).

### Beispiel 6

Die gesamte Menge des in Beispiel 2 erhaltenen Phospholipidgels (50 g) wird mit 84 g Leitungswasser versetzt, 4 Min. verrührt und dann 16 g Ethanol zugegeben. Nach einer weiteren Rührzeit von 1 Min. erhält man als Endprodukt eine liposomale Lösung mit einem Ethanolgehalt von 16 Gew.% und 10,48 Gew.% Phospholipidkonzentrat (entsprechend 10 Gew.% Phospholipiden) und einer mittleren Teilchengröße von 194 nm (± 20%). Trotz der Verwendung von Leitungswasser, das üblicherweise mit Mikroorganismen und Salzen verunreinigt ist, erhält das Produkt weniger als 100 Keime pro Gramm.

### Beispiel 7

Analog Beispiel 6 werden der gesamten Gelmenge aus Beispiel 4 111 g physiologische Kochsalzlösung (0,9 Gew.% Natriumchlorid) zugegeben. Nach 4 Min. Rühren gibt man noch 24 g 2-Propanol hinzu und rührt eine weitere Minute. Die mittlere Teilchengröße der Vesikel in der liposomalen Lösung beträgt 200 nm (± 20%).

### Beispiel 8

Das in Beispiel 4 erhaltene Phospholipidgel wird unter Rühren mit 37 g 0.2 molarer Phosphatpufferlösung versetzt, 4 Min. gerührt, 8 g 2-Propanol hinzugegeben und noch 1 Min. weitergerührt. Die mittlere Teilchengröße der Vesikel in der liposomalen Lösung beträgt 187 nm (± 20%).

Die Keimzahl der erfindungsgemäßen gelartigen Phospholipidzusammensetzungen gemäß Beispielen 1 bis 4 sowie der erfindungsgemäßen hieraus erhaltenen liposomalen Lösungen nach Beispielen 5 bis 8 wurde nach den Anforderungen des DAB 9 für Arzneimittel der Kategorie 2, Zubereitungen zur topischen oder andersartigen lokalen Anwendung bestimmt. In allen Fällen lag die Keimzahl unter 100 Keimen/g der Zubereitung und entspricht damit den DAB 9 Anforderungen.

### Zubereitungen mit biologisch aktiven Wirkstoffen

Die Liposomen, die im erfindungsgemäß hergestellten Gel vorhanden sind (Fig. 1), können mit verschiedenen Wirksubstanzen beladen werden. Überraschenderweise kann die Beladung sowohl mit lipophilen (z.B. Bisabolol) als auch mit hydrophilen (z.B. Papaverin x HCl) Substanzen vorgenommen werden.

### Zubereitung 1:

97,0 g erfindungsgemäß hergestelltes Gel gemäß Beispiel 1 werden mit 3,0 g Bisabolol mittels eines Propellerrührers 10 Min bei 50°C gerührt. 15,0 g dieses Gemisches werden mit 85,00 g entmineralisiertem Wasser verdünnt. Die mittlere Teilchengröße einer mit entmineralisiertem Wasser auf 0,01% Phospholipid verdünnten Lösung betrug 215 nm (Laserlichtstreuung).

### Zubereitung 2:

4,0 g Papaverin *HCl werden in 36.0 g Ethanol gelöst und mit 360,0 g erfindungsgemäß hergestelltem Gel gemäß Beispiel 1 in einem schnell rührenden Mixer homogenisiert. Die mittlere Teilchengröße betrug 175 nm (Laserlichtstreuung).

### Zubereitung 3:

1,43 g Hirudin (100.000 ATE/100 g) werden mit 98,57 g des erfindungsgemäß hergestellten Gels gemäß Beispiel 1 in einer Fantaschale verrührt und in einem schnell rührenden Mixer homogenisiert. Die mittlere Teilchengröße betrug 151 nm (Laserlichtstreuung), der pH-Wert 6,9.

### Zubereitung 4:

1,98 g Heparin-Na werden in 40,0 g Ethanol, 204,33 g entmineralisiertem Wasser und 3,7 g NaCl mittels eines Magnetrührers gelöst. Diese Lösung wird mit 250,0 g erfindungsgemäß hergestellten Gels gemäß Beispiel 1 mit einem schnell rührenden Mixer homogenisiert. Die mittlere Teilchengröße einer mit entmineralisiertem Wasser auf 0,01% Phospholipid verdünnten Lösung betrug 231 nm, der pH-Wert 6,5.

### Zubereitung 5:

1,0 g Ketoprofen werden mit 1,60 g Ethanol, 90,0 g erfindungsgemäß hergestelltem Gel gemäß Beispiel 1, 8,4 g entmineralisiertem Wasser und 0,60 g 10 %-iger wäßriger Natriumhydroxidlösung in einer Fantaschale verrührt. Die mittlere Teilchengröße einer mit entmineralisiertem Wasser auf 0,01 % Phospholipid verdünnten Lösung betrug 216 nm.

### Zubereitung 6:

5 g Harnstoff werden mit 20 g des gemäß Beispiel 1 hergestellten Gels verrührt und anschließend mit entmineralisiertem Wasser auf 3% Phospholipid verdünnt. Es entstehen Liposomen mit einer mittleren Teilchengröße von 175 nm.

### Zubereitung 7:

5 g Elastin werden mit 20 g des gemäß Beispiel 1 hergestellen Gels verrührt und anschließend mit entmineralisiertem Wasser auf 3% Phospholipid verdünnt. Es entstehen Liposomen mit einer mittleren Teilchengröße von 171 nm.

## Patentansprüche

1. Alkohol enthaltende wäßrige gelartige Phospholipidzusammensetzung, die als Alkohole Ethanol, 1-Propanol, 2-Propanol oder deren Mischungen enthält,
**dadurch gekennzeichnet,**
daß diese Zusammensetzung ein liposomales Gel mit folgenden Bestandteilen ist:
- 15,00 - 30,00 Gew.Tl. eines Phospholipidkonzentrats, bestehend aus
70,00 - 80,00 Gew.Tl. Phosphatidylcholin,
15,00 - 5,00 Gew.Tl. sauren Phospholipiden,
5,00 - 25,00 Gew.Tl. sonstigen Phospholipiden,
wobei dieses Konzentrat pro 100 Gew.Tl. vorstehender Phospholipide weiterhin 1-15 Gew.Tl. phosphorfreie Begleitlipide enthält,
- 20,00 - 14,00 Gew.Tl. Alkohol sowie
- 50,00 - 71,00 Gew.Tl. einer wäßrigen Lösung.

2. Phospholipidzusammensetzung nach Anspruch 1,
**dadurch gekennzeichnet,**
daß das Phospholipidkonzentrat aus
80 Gew.Tl. Phosphatidylcholin,
5 - 15 Gew.Tl. sauren Phospholipiden,
15 - 5 Gew.Tl. sonstigen Phospholipiden
besteht,
wobei dieses Konzentrat pro 100 Gew.Tl. vorstehender Phospholipide weiterhin 1 - 9 Gew.Tl. phosphorfreie Begleitlipide enthält.

3. Phospholipidzusammensetzung nach Anspruch 1 und 2,
**dadurch gekennzeichnet,**
daß das liposomale Gel einen Alkoholgehalt in der Endformulierung von etwa 16 Gew.% aufweist.

4. Verwendung der Phospholipidzusammensetzung nach Ansprüchen 1 bis 3, zur Herstellung von liposomalen Lösungen durch Verdünnen mit einer Wasser enthaltenden Lösung.

5. Verwendung nach Anspruch 4,
**dadurch gekennzeichnet,**
daß die Wasser enthaltende Lösung weiterhin wenigstens einen biologisch aktiven Wirkstoff enthält.

6. Verwendung nach Anspruch 5,
**dadurch gekennzeichnet,**
daß der Wirkstoff ein Antiphlogistikum, ein Anticoagulanz, ein Antimykotikum, ein Spasmolytikum oder ein durchblutungsförderndes Mittel oder ein in die Hornhaut penetrierendes pflegendes Mittel ist.

7. Topisch applizierbare pharmazeutische oder kosmetische Zubereitungen, enthaltend wenigstens eine Phospholipidzusammensetzung nach Ansprüchen 1 bis 3 in Kombination mit wenigstens einem biologisch aktiven Wirkstoff und üblichen Hilfs- und Zusatzstoffen.

8. Zubereitungen nach Anspruch 7,
**dadurch gekennzeichnet**,
daß der Wirkstoff ein Antiphlogistikum, ein Anticoagulanz, ein Antimykotikum, ein Spasmolytikum, ein durchblutungsförderndes Mittel oder ein in die Hornhaut penetrierendes pflegendes Mittel ist.

## Claims

1. Alcoholic, aqueous gel-like phospholipid composition which, as alcohols, contains ethanol, 1-propanol, 2-propanol or mixtures thereof, characterized in that this composition is a liposomal gel containing the following constituents:
- 15.00-30.00 parts by weight of a phospholipid concentrate, consisting of
70.00-80.00 parts by weight of phosphatidylcholine,
15.00- 5.00 parts by weight of acidic phospholipids,
5.00-25.00 parts by weight of other phospholipids,
this concentrate additionally containing 1-15 parts by weight of phosphorus-free associated lipids per 100 parts by weight of the above phospholipids,
- 20.00-14.00 parts by weight of alcohol and
- 50.00-71.00 parts by weight of an aqueous solution.

2. Phospholipid composition according to Claim 1, characterized in that the phospholipid concentrate consists of
80 parts by weight of phosphatidylcholine,
5-15 parts by weight of acidic phospholipids,
15- 5 parts by weight of other phospholipids,
this concentrate furthermore containing 1-9 parts by weight of phosphorus-free associated lipids per 100 parts by weight of the above phospholipids.

3. Phospholipid composition according to Claim 1 and 2, characterized in that the liposomal gel has an alcohol content in the final formulation of about 16% by weight.

4. Use of the phospholipid composition according to Claims 1 to 3 for the preparation of liposomal solutions by dilution with a water-containing solution.

5. Use according to Claim 4, characterized in that the water-containing solution additionally contains at least one biologically active substance.

6. Use according to Claim 5, characterized in that the active substance is an anti-inflammatory, an anticoagulant, an antimycotic, a spasmolytic or a circulation-promoting agent or a caring agent penetrating into the horny skin.

7. Topical pharmaceutical or cosmetic preparations containing at least one phospholipid composition according to Claims 1 to 3 in combination with at least one biologically active substance and customary auxiliaries and additives.

8. Preparations according to Claim 7, characterized in that the active substance is an anti-inflammatory, an anticoagulant, an antimycotic, a spasmolytic, a circulation-promoting agent or a caring agent penetrating into the horny skin.

## Revendications

1. Composition phospholipidique aqueuse sous forme de gel contenant de l'alcool, qui contient comme alcool de l'éthanol, du propanol-1, du propanol-2 ou des mélanges de ceux-ci, caractérisée en ce qu'elle est constituée d'un gel liposomal comprenant les constituants suivants :
- 15,00 à 30,00 parties en poids d'un concentrat de phospholipides constitué:
70,00 à 80,00 parties en poids de phosphatidylcholine,
15,00 à 5,00 parties en poids de phospholipides acides,
5,00 à 25,00 parties en poids d'autres phospholipides,
ce concentrat contenant en outre, pour 100 parties en poids des phospholipides précédents, 1 à 15 parties en poids de lipides supplémentaires exempts de phosphore,
- 20,00 à 14,00 parties en poids d'alcool et
- 50,00 à 71,00 parties en poids d'une solution aqueuse.

2. Composition phospholipidique selon la revendication 1, caractérisée en ce que le concentrat phospholipidique est constitué de :
80 parties en poids de phosphatidylcholine,
5 à 15 parties en poids de phospholipides acides,
15 à 5 parties en poids d'autres phospholipides,
ce concentrat contenant en outre 1 à 9 parties en poids de lipides supplémentaires exempts de phosphore pour 100 parties en poids des phospholipides précédents.

3. Composition phospholipidique selon la revendication 1 et 2, caractérisée en ce que le gel liposomal possède une teneur en alcool dans la composition finale qui est égale à 16 % en poids environ.

4. Application de la composition phospholipidique selon les revendications 1 à 3 à la préparation de solutions liposomales par dilution avec une solution contenant de l'eau.

5. Application selon la revendication 4, caractérisée en ce que la solution contenant de l'eau contient en outre au moins une substance biologiquement active.

6. Application selon la revendication 5, caractérisée en ce que la substance active est un anti-inflammatoire, un anticoagulant, un antifongique, un spasmolytique, un agent favorisant la circulation ou un agent de soins pénétrant dans la couche cornée.

7. Préparations pharmaceutiques ou cosmétiques pouvant être appliquées par voie topique, contenant au moins une composition phospholipidique selon les revendications 1 à 3 en combinaison avec au moins une substance biologiquement active et des additifs et adjuvants usuels.

8. Préparations selon la revendication 7, caractérisées en ce que la substance active est un anti-inflammatoire, un anticoagulant, un antifongique, un spasmolytique, un agent favorisant la circulation ou un agent de soins pénétrant dans la couche cornée.
